# EUROPEAN PATENT SPECIFICATION

(11) **EP 0 971 729 B1**
(45) Date of publication and mention of the grant of the patent: **03.07.2002**
(21) Application number: 98907915.7
(22) Date of filing: 20.03.1998
(51) Int. Cl.: A61K 38/28

(54) **THERAPEUTIC POWDER FORMULATION FOR PULMONARY ADMINISTRATION, CONTAINING CRYSTALLINE INSULIN**
THERAPEUTISCHE PULVERFORMULIERUNG ZUR PULMONAREN ANWENDUNG, WELCHE KRISTALLINES INSULIN ENTHÄLT
FORMULATION DE POUDRE THERAPEUTIQUE DESTINEE A ETRE ADMINISTREE DANS LES VOIES RESPIRATOIRES ET CONTENANT DE L'INSULINE CRISTALLINE

(30) Priority: 20.03.1997 DK 31997
(43) Date of publication of application: 19.01.2000
(73) Proprietor: NOVO NORDISK A/S, 2880 Bagsvaerd (DK)
(72) Inventor: JENSEN, Steen, DK-2791 Drag r (DK); HANSEN, Philip, DK-2840 Holte (DK)
(74) Representative: Kellberg, Lars
(86) International application number: DK9800108
(87) International publication number: WO9842368

(56) References cited:
- EP-A- 0 025 868
- EP-A- 0 709 395
- WO-A-95/00128
- WO-A-96/19207

## Description

### Field of the invention

The present invention relates to a therapeutic powder formulation suitable for pulmonary administration comprising particles composed of human insulin or any analogue or derivative thereof and a an enhancer which enhances the absorption of insulin in the lower respiratory tract.

### Background of the invention

Diabetes is a general term for disorders in man having excessive urine excretion as in diabetes mellitus and diabetes insipidus. Diabetes mellitus is a metabolic disorder in which the ability to utilize glucose is more or less completely lost. About 2 % of all people suffer from diabetes.

Since the introduction of insulin in the 1920's, continuous strides have been made to improve the treatment of diabetes mellitus. To help avoid extreme glycaemia levels, diabetic patients often practice multiple injection therapy, whereby insulin is administered with each meal.

In the treatment of diabetes mellitus, many varieties of insulin preparations have been suggested and used, such as regular insulin, Semilente® insulin, isophane insulin, insulin zinc suspensions, protamine zinc insulin and Ultralente® insulin. Some of the commercial available insulin preparations are characterized by a fast onset of action. Ideally, exogenous insulin is administered at times and in doses that would yield a plasma profile which mimics the plasma profile of endogenous insulin in a normal individual. Insulin preparations containing analogs of human insulin have shown an absorption profile very close to the normal plasma profile, e.g. Lys^{B28}-Pro^{B29} human insulin and Asp^{B28} human insulin. However, these new insulin preparations still has to be injected in connection with a meal. In order to circumvent injections, administration of insulin via the pulmonary route could be an alternative elucidating absorption profiles which mimic the endogenous insulin without the need to inject the insulin.

### Description of the background art

Administration of insulin via the pulmonary route can be accomplished by either an aqueous solution or a powder preparation. A description of the details can be found in several references, one of the latest being by Niven, Crit. Rev. Ther. Drug Carrier Sys, 12(2&3):151-231 (1995). One aspect covered in said review is the stability issue of protein formulations, aqueous solutions being less stable than powder formulation. So far, all powder formulations have been described as mainly amorphous.

It has been found that when insulin is combined with an appropriate absorption enhancer and is introduced into the lower respiratory tract in the form of a powder of appropriate particle size, it readily enters the systemic circulation by absorption through the layer of epithelial cells in the lower respiratory tract as described in US patent No. 5,506,203. The manufacturing process described in said patent, comprising dissolution of insulin at acid pH followed by a pH adjustment to pH 7.4 and addition of sodium taurocholate before drying the solution by a suitable method, results in a powder composed of human insulin and absorption enhancer in a ratio between 9:1 to about 1:1. The powder is characterized as mainly amorphous determined under a polarized light microscope.

WO 96/19207 relates to a powder formulation of medically useful polypeptides such as insulin. The powder formulation is characterised by containing melezitose as diluent, and can furthermore comprise an enhancer which enhances the absorption of the polypeptide in the lower respiratory tract such as sodium taurocholate. Two different general methods for producing powder formulations are disclosed in this document. The first method involves dry mixing of the components as well as micronisation. The second method involves dissolution of the components in a suitable solvent (such as water) and, if desired, adjustment of pH. The powder is then obtained by removing the water by a suitable drying method (i.e. forced precipitation) such as vacuum concentration, open drying, spray drying, and freeze drying.

WO 95/00128 is concerned with dry powder compositions comprising a pharmaceutically active polypeptide and an enhancer which enhances the absorption in the lower respiratory tract. The polypeptide can be a wide range of peptide hormones, but the polypeptide is preferably not insulin.

### Description of the invention

### Definitions

The expressions "crystalline" and "amorphous" as used herein corresponds to different states of a powder particle, distinguishable by the following method: An aliquot of particles of the powder are mounted in mineral oil on a clean glass slide. Examination using a polarized light microscope elucidates birefringence for crystalline particles.

The expression "enhancer" refers to a substance enhancing the absorption of insulin, insulin analogue or insulin derivative through the layer of epithelial cells lining the alveoli of the lung into the adjacent pulmonary vasculature, i.e. the amount of insulin absorbed into the systemic system is higher than the amount absorbed in the absence of enhancer.

By "analogue of human insulin" (or similar expressions) as used herein is meant human insulin in which one or more amino acids have been deleted and/or replaced by other amino acids, including non-codeable amino acids, or human insulin comprising additional amino acids, i.e. more than 51 amino acids.

By "derivative of human insulin" (or similar expressions) as used herein is meant human insulin or an analogue thereof in which at least one organic substituent is bound to one or more of the amino acids.

In the present context the expression "powder" refers to a collection of essentially dry particles, i.e. the moisture content being below about 10 % by weight, preferably below 6 % by weight, and most preferably below 4 % by weight.

The present invention relates to a therapeutic powder formulation suitable for pulmonary administration comprising particles composed of human insulin or any analogue or derivative thereof and an enhancer which enhances the absorption of insulin in the lower respiratory tract, wherein at least 50% by weight of said particles are crystalline.

The crystalline powder formulation of insulin and enhancer elucidates a better stability profile than powders of essentially the same composition prepared by spray drying, freeze-drying, vacuum drying and open drying. This is probably due to the substantially crystalline state of the powder formulations of the present invention compared to the amorphous state of powders prepared by the other methods described. By this means it is possible to store the powder formulations of the present invention at room temperature in contrary to human insulin preparations for injections and some amorphous insulin powders without stabilizing agent which have to be stored between 2°C to 8°C.

Furthermore, the substantially crystalline powder formulation of insulin and enhancer elucidates better flowing properties than corresponding amorphous powder formulations.

Preferably, at least 75% by weight, more preferably at least 90% by weight, of said particles are crystalline.

The enhancer is advantageously a surfactant, preferably selected from the group consisting of salts of fatty acids, bile salts or phospholipids, more preferably a bile salt.

Preferred fatty acids salts are salts of C₁₀₋₁₄ fatty acids, such as sodium caprate, sodium laurate and sodium myristate.

Lysophosphatidylcholine is a preferred phospholipid.

Preferred bile salts are salts of ursodeoxycholate, taurocholate, glycocholate and taurodihydrofusidate. Still more preferred are powder formulations according to the invention wherein the enhancer is a salt of taurocholate, preferably sodium taurocholate.

The preferred analogues of human insulin are fast-acting insulin analogues, in particular analogues wherein position B28 is Asp, Lys, Leu, Val or Ala and position B29 is Lys or Pro; or des(B28-B30), des(B27) or des(B30) human insulin. The most preferred analogues are Asp^{B28} human insulin or Lys^{B28}Pro^{B29} human insulin.

The preferred derivatives of human insulin are derivatives comprising one or more lipophilic substituents. The preferred lipophilic insulins are acylated insulins such as those described in WO 95/07931, e.g. human insulin derivatives wherein the ε-amino group of Lys^{B29} contains an acyl substituent which comprises at least 6 carbon atoms.

The insulin derivative is most preferably selected from the group consisting of B29-N^{ε}-myristoyl-des(B30) human insulin, B29-N^{ε}-palmitoyl-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-palmitoyl Lys^{B28} Pro^{B29} human insulin, B30-N^{ε}-myristoyl-Thr^{B29}Lys^{B30} human insulin, B30-N^{ε}-palmitoyl-Thr^{B29}Lys^{B30} human insulin, B29-N^{ε}-(N-palmitoyl-γ-glutamyl)-des(B30) human insulin, B29-N^{ε}-(N-lithocholyl-γ-glutamyl)-des(B30) human insulin, B29-N^{ε}-(ω-carboxyheptadecanoyl)-des(B30) human insulin and B29-N^{ε}-(ω-carboxyheptadecanoyl) human insulin.

In a preferred embodiment the powder formulation of the present invention comprises an insulin derivative as well as human insulin or an analogue thereof.

However, human insulin is the most preferred insulin to be used in the formulation of the present invention.

In a particular embodiment of the present invention the powder formulation further comprises zinc, preferably in an amount corresponding to 2 Zn atoms/insulin hexamer to 12 Zn atoms/insulin hexamer, more preferably 4 Zn atoms/insulin hexamer to 12 Zn atoms/insulin hexamer or 2 Zn atoms/insulin hexamer to 10 Zn atoms/insulin hexamer, still more preferably 2 Zn atoms/insulin hexamer to 5 Zn atoms/insulin hexamer. By means of adding zinc to the preparations it is possible to adjust the timing, i.e. obtain the desired biological response within a defined time span, of the formulation as preparations with 0 - 10 Zn atoms/insulin hexamer elucidates different solubility properties determined in vitro with a 7 mM phosphate buffer solution.

In a preferred embodiment of the present invention, the major part of the crystals of the powder formulation have a maximum diameter of up to 10 µm, preferably up to 7.5 µm, more preferably up to 5 µm. Powder formulations in which at least 80% or essentially all crystals have a maximum diameter within the above range are most preferred.

The molar ratio of insulin to enhancer in the powder formulation of the present invention is preferably 9:1 to 1:9, more preferably between 5:1 to 1:5, and still more preferably between 3:1 to 1:3.

The powder formulations of the present invention may optionally be combined with a carrier or excipient generally accepted as suitable for pulmonary administration. The purpose of adding a carrier or excipient may be as a bulking agent, stabilizing agent or an agent improving the flowing properties.

Suitable carrier agents include 1) carbohydrates, e.g. monosaccharides such as fructose, galactose, glucose, sorbose, and the like; 2) disaccharides, such as lactose, trehalose and the like; 3) polysaccharides, such as raffinose, maltodextrins, dextrans, and the like; 4) alditols, such as mannitol, xylitol, and the like; 5) inorganic salts, such as sodium chloride, and the like; 6) organic salts, such as sodium citrate, sodium ascorbate, and the like. A preferred group of carriers includes trehalose, raffinose, mannitol, sorbitol, xylitol, inositol, sucrose, sodium chloride and sodium citrate.

In a preferred embodiment the therapeutic powder formulation according to the invention comprises a stabilizing amount of a phenolic compound, preferably in an amount corresponding to at least 3 molecules of phenolic compound/insulin hexamer. The phenolic compound is preferably phenol, m-cresol, or a mixture of these compounds.

The powder formulation of the present invention may be produced according to the following general procedure:

Crystallization of insulin and the enhancer is accomplished by dissolving insulin in a dilute acidic solution, e.g. at a pH = 3.0 - 3.9, optionally adding a desired amount of zinc, e.g. corresponding to preferably 2 Zn atoms/insulin hexamer to 10 Zn atoms/insulin hexamer, more preferably 2 Zn atoms/insulin hexamer to 5 Zn atoms/insulin hexamer. Finally, the insulin/zinc solution is mixed under slight agitation with a solution of the enhancer. The proportion of insulin and enhancer on a weight basis when mixing the solutions is preferably between 9:1 to 1:9, more preferably between 5:1 to 1:5, and still more preferably between 3:1 to 1:3. The pH of the preparation is then adjusted to a value in the range of 4.5 to 7.4, preferably 4.5 to 7, more preferably 4.5 to 6.5, still more preferably 5.5 to 6.2, most preferably 5.5 to 6.1, and allowed to stand at rest for approximately 16 hours at a temperature between 20°C to 34°C, more preferable between 20°C to 25°C. The crystals formed are isolated by vacuum evaporation. The insulin powder can, if necessary, be micronized.

This invention is further illustrated by the following examples which, however, are not to be construed as limiting.

### EXAMPLE I

249.8 mg human insulin was dissolved in water by adding 2N HCI resulting in a pH = 3.7-3.8. 50 µL 4 % Zinc chloride solution was added to the insulin solution while mixing. Water was added to 10 mL. 1g sodium taurocholate was dissolved in 10 mL water. Another insulin solution without the addition of zinc chloride was prepared by dissolving 251.6 mg human insulin in water by adding 2N HCI resulting in a pH = 3.6 - 3.7.

To three beakers were added 400µL, 450 µL and 500 µL, respectively, of the sodium taurocholate solution. 1.6 mL of the insulin solution containing zinc chloride was then added to each beaker while mixing. Water ad 10 mL was finally added while mixing.

To three other beakers were added 400µL, 450 µL and 500 µL, respectively, of the sodium taurocholate solution. 1.6 mL of the insulin solution without zinc chloride was then added to each beaker while mixing. Water ad 10 mL was finally added while mixing.

The pH was adjusted to 6.1 while mixing in all six beakers. After standing at rest for approximately 16 hours at 20°C - 25°C, crystals were formed in all preparations.

An aliquot of each preparation elucidates almost complete crystalline state of the particles as determined under a polarized light microscope. The size of the individual crystals was determined to 1µm - 5µm.

The supernatant was carefully removed from each of the preparations and the remaining wet crystalline fraction was dried by placing in a vacuumdryer for approximately 5 hours.

The dry insulin powders were analyzed by RP-HPLC for the content of human insulin and sodium taurocholate and the results showed a proportion of human insulin and sodium taurocholate of 4:1 to 2:1 depending on the content of sodium taurocholate. No difference was observed between the preparations with and without zinc chloride

### EXAMPLE II

625.9 mg human insulin was dissolved in water by adding 2N HCI resulting in a pH = 3.6-3.7. 125 µL 4 % Zinc chloride solution was added to the insulin solutions while mixing.

Water was added to 25 mL. 1g sodium taurocholate was dissolved in 10 mL water. To 16 mL of the insulin solution was then added 4 mL of the taurocholate solution while mixing. Water ad 100 mL was finally added while mixing. The preparation with the spontaneous amorphous precipitate was divided in 7 beakers with 10 mL in each. The pH was adjusted to 4.5, 5.0, 5.5, 6.0, 6.1, 6.5, 7.0 and 7.4 while mixing. After standing at rest for approximately 16 hours at 20°C - 25°C, crystals were formed in all preparations.

An aliquot of each preparation elucidates almost complete crystalline state of the particles as determined under a polarized light microscope. The size of the individual crystals was determined to 1µm - 5µm.

The supernatant was carefully removed from each of the preparations and the remaining wet crystalline fraction was dried by placing in a vacuumdryer for approximately 5 hours.

The dry insulin powders were analyzed by RP-HPLC for the content of human insulin and sodium taurocholate and the results showed a proportion of human insulin and sodium taurocholate of 6:1 to 3:1 depending on the actual pH value

### EXAMPLE III

625.9 mg human insulin was dissolved in water by adding 2N HCI resulting in a pH = 3.6-3.7. Water was added to 25 mL. 1g sodium taurocholate was dissolved in 10 mL water. The insulin solution was divided in 5 beakers with 4 mL in each. A 0.4 % Zinc chloride solution was added to the insulin solutions while mixing in an increasing amount: 81 µL, 123 µL, 164µL, 205µL, 285µL and 410µL. To each of the solutions were then added 1 mL of the taurocholate solution while mixing. Water ad 25 mL was finally added while mixing. The pH was adjusted to 6.1 while mixing. Spontaneously, an amorphous precipitate was formed in each of the preparations. After standing at rest for approximately 16 hours at 20°C - 25°C, crystals were formed in all preparations.

An aliquot of each preparation elucidates almost complete crystalline state of the particles as determined under a polarized light microscope. The size of the individual crystals was determined to 1µm - 5µm.

The supernatant was carefully removed from each of the preparations and the remaining wet crystalline fraction was dried by placing in a vacuumdryer for approximately 5 hours.

The dry insulin powders were analyzed by RP-HPLC for the content of human insulin and sodium taurocholate and the results showed a proportion of human insulin and sodium taurocholate of 6:1 to 4:1depending on the content of zinc.

### EXAMPLE IV

625,3 mg human insulin was dissolved in water by adding 2N HCI resulting in a pH= 3.6-3.7. 125µL 4 % Zinc chloride solution was added to the insulin solution while mixing. Water was added to 25 mL. 1 g sodium taurocholate was dissolved in 10 mL water. The insulin solution was divided in 4 beakers with 1.6 mL in each. To each of the beakers were added 400 µL of taurocholate solution while mixing. A sodium chloride solution (100 mg/mL) was added while mixing in an increasing amount: 0 µL, 58µL, 116µL and 232µL.Water ad 10 mL was finally added while mixing. The pH was adjusted to 6.1 while mixing.

An aliquot of each preparation elucidates 50% to 80% crystalline state of the particles as determined under a polarized light microscope. The size of the individual crystals was determined to 1µm - 5µm.

The dry insulin powders were analyzed for the content of human insulin and sodium taurocholate and the results showed a proportion of human insulin and sodium taurocholate of 3:1 in all the preparations

### EXAMPLE V

2.5 g human insulin was dissolved in water by adding 2N HCI resulting in a pH = 3.6 - 3.7. 500 µL 4 % Zinc chloride solution was added to the insulin solutions while mixing. Water was added to 100 mL. 2.5 g sodium taurocholate was dissolved in 25 mL water. The insulin solution was divided in 9 beakers with 8 mL in each. To 3 insulin solutions (group 1) were added 2 mL, to the next 3 insulin solutions (group 2) were added 2.25 mL and to the last 3 insulin solutions (group 3) were added 2.50 mL of the taurocholate solution while mixing. In each of the 3 groups, a sodium chloride solution 100 mg/mL was added in increasing amounts: 0 µL, 290 µL and 1160 µL. Water ad 50 mL was finally added while mixing. The pH was adjusted to 6.1 while mixing. Spontaneously, an amorphous precipitate was formed in each of the preparations. After standing at rest for approximately 16 hours at 20°C - 25°C, crystals were formed in all preparations.

An aliquot of each preparation elucidates almost complete crystalline state of the particles with no sodium chloride added while the preparations with sodium chloride elucidate approximately 50 % to 80 % crystalline state as determined under a polarized light microscope. The size of the individual crystals was determined to 1µm - 5µm.

The supernatant was carefully removed from each of the preparations and the remaining wet crystalline fraction was dried by placing in a vacuum dryer for approximately 5 hours.

The dry insulin powders were analyzed for the content of human insulin and sodium taurocholate and the results showed a proportion of human insulin and sodium taurocholate of 6:1 to 3:1 in the preparations.

## Claims

1. A therapeutic powder formulation suitable for pulmonary administration comprising particles composed of human insulin or any analogue or derivative thereof and an enhancer which enhances the absorption of insulin in the lower respiratory tract, wherein at least 50% by weight of said particles are crystalline.

2. A therapeutic powder formulation according to claim 1 wherein at least 75% by weight, preferably at least 90% by weight, of said particles are crystalline.

3. A therapeutic powder formulation according to claim 1 or 2 wherein the enhancer is a surfactant.

4. A therapeutic powder formulation according to claim 3 wherein the surfactant is a salt of a fatty acid, a bile salt or a phospholipid, preferably a bile salt.

5. A therapeutic powder formulation according to claim 4 wherein the surfactant is a salt of taurocholate, preferably sodium taurocholate.

6. A therapeutic powder formulation according to anyone of the preceding claims which further comprises zinc in an amount corresponding to 2 Zn atoms/insulin hexamer to 12 Zn atoms/insulin hexamer, preferably 4 Zn atoms/insulin hexamer to 12 Zn atoms/insulin hexamer.

7. A therapeutic powder formulation according to claim 6 which comprises zinc in an amount corresponding to 2 Zn atoms/insulin hexamer to 10 Zn atoms/insulin hexamer, preferably 2 Zn atoms/insulin hexamer to 5 Zn atoms/insulin hexamer.

8. A therapeutic powder formulation according to anyone of the preceding claims wherein the major part of the crystals have a maximum diameter of up to 10 µm, preferably up to 7.5 µm.

9. A therapeutic powder formulation according to anyone of the preceding claims wherein the molar ratio of insulin to enhancer is 9:1 to 1:9, preferably between 5:1 to 1:5, and more preferably between 3:1 to 1:3.

10. A therapeutic powder formulation according to anyone of the preceding claims which further comprises a carrier, preferably selected from the group consisting of trehalose, raffinose, mannitol, sorbitol, xylitol, inositol, sucrose, sodium chloride and sodium citrate.

11. A therapeutic powder formulation according to anyone of the preceding claims which further comprises a stabilizing amount of a phenolic compound.

12. A therapeutic powder formulation according to claim 11 which comprises at least 3 molecules of a phenolic compound/insulin hexamer.

13. A therapeutic powder formulation according to claim 11 or 12 which comprises m-cresol or phenol, or a mixture thereof.

## Patentansprüche

1. Therapeutische Pulverformulierung geeignet zur Lungenverabreichung, umfassend Partikel bestehend aus Humaninsulin oder einem Analog oder Derivat davon, und einem Verstärker, der die Absorption von Insulin in dem unteren Atemtrakt verstärkt, wobei wenigstens 50 Gew% der Partikel kristallin sind.

2. Therapeutische Pulverformulierung nach Anspruch 1, wobei wenigstens 75 Gew%, vorzugsweise wenigstens 90 Gew% der Partikel kristallin sind.

3. Therapeutische Pulverformulierung nach Anspruch 1 oder 2, wobei der Verstärker ein oberflächenaktiver Stoff ist.

4. Therapeutische Pulverformulierung nach Anspruch 3, wobei der oberflächenaktive Stoff ein Salz oder eine Fettsäure, ein Gallensalz oder ein Phospholipid, vorzugsweise ein Gallensalz ist.

5. Therapeutische Pulverformulierung nach Anspruch 4, wobei der oberflächenaktive Stoff ein Salz von Taurocholat, vorzugsweise Natriumtaurocholat ist.

6. Therapeutische Pulverformulierung nach einem der vorhergehenden Ansprüche, die weiterhin Zink in einer Menge entsprechend 2 Zn Atomen/Insulinhexamer bis 12 Zn Atomen/Insulinhexamer, vorzugsweise 4 Zn Atomen/Insulinhexamer bis 12 Zn Atomen/Insulinhexamer aufweist.

7. Therapeutische Pulverformulierung nach Anspruch 6, die Zink in einer Menge entsprechend 2 Zn Atomen/Insulinhexamer bis 10 Zn Atomen/Insulinhexamer, vorzugsweise 2 Zn Atomen/Insulinhexamer bis 5 Zn Atomen/Insulinhexamer aufweist.

8. Therapeutische Pulverformulierung nach einem der vorhergehenden Ansprüche, wobei der Großteil der Kristalle einen maximalen Durchmesser von bis zu 10 µm, vorzugsweise bis zu 7,5 µm aufweist.

9. Therapeutische Pulverformulierung nach einem der vorhergehenden Ansprüche, wobei das molare Verhältnis von Insulin zu Verstärker 9:1 bis 1:9, vorzugsweise zwischen 5:1 bis 1:5, und weiter bevorzugt zwischen 3:1 bis 1:3 beträgt.

10. Therapeutische Pulverformulierung nach einem der vorhergehenden Ansprüche, die weiterhin einen Träger aufweist, der vorzugsweise ausgewählt ist aus der Gruppe bestehend aus Trehalose, Raffinose, Mannitol, Sorbitol, Xylitol, Inositol, Sucrose, Natriumchlorid und Natriumcitrat.

11. Therapeutische Pulverformulierung nach einem der vorhergehenden Ansprüche, die weiterhin eine stabilisierende Menge einer phenolischen Verbindung aufweist.

12. Therapeutische Pulverformulierung nach Anspruch 11, die wenigstens 3 Moleküle einer phenolischen Verbindung/Insulinhexamer aufweist.

13. Therapeutische Pulverformulierung nach Anspruch 11 oder 12, die m-Cresol oder Phenol oder ein Mischung davon aufweist.

## Revendications

1. Une formulation de poudre thérapeutique appropriée pour l'administration pulmonaire, comprenant des particules composées d'insuline humaine ou de n'importe quel analogue ou dérivé de celle-ci et d'un activateur qui active l'absorption d'insuline dans la voie respiratoire inférieure, dans laquelle au moins 50% en poids desdites particules sont cristallines.

2. Une formulation de poudre thérapeutique selon la revendication 1, dans laquelle au moins 75% en poids, de préférence au moins 90% en poids desdites particules sont cristallines.

3. Une formulation de poudre thérapeutique selon la revendication 1 ou 2, dans laquelle l'activateur est un tensioactif.

4. Une formulation de poudre thérapeutique selon la revendication 3, dans laquelle le tensioactif est un sel d'un acide gras. un sel de la bile ou un phospholipide, de préférence un sel de la bile.

5. Une formulation de poudre thérapeutique selon la revendication 4, dans laquelle le tensioactif est un sel de taurocholate, de préférence du taurocholate de sodium.

6. Une formulation de poudre thérapeutique selon l'une quelconque des revendications précédentes, qui comprend, en outre, du zinc selon une quantité correspondant à 2 atomes de Zn/hexamère d'insuline à 12 atomes de Zn/hexamère d'insuline, de préférence à 4 atomes de Zn/hexamère d'insuline à 12 atomes de Zn/hexamère d'insuline.

7. Une formulation de poudre thérapeutique selon la revendication 6, qui comprend du zinc selon une quantité correspondant à 2 atomes de Zn/hexamère d'insuline à 10 atomes de Zn/hexamère d'insuline, de préférence à 2 atomes de Zn/hexamère d'insuline à 5 atomes de Zn/hexamère d'insuline.

8. Une formulation de poudre thérapeutique selon l'une quelconque des revendications précédentes, dans laquelle la majeure partie des cristaux présente un diamètre maximum jusqu'à 10 µm, de préférence jusqu'à 7,5 µm.

9. Une formulation de poudre thérapeutique selon l'une quelconque des revendications précédentes, dans laquelle le rapport molaire de l'insuline à l'activateur est de 9:1 à 1:9, de préférence compris entre 5:1 et 1:5, mieux encore compris entre 3:1 et 1:3.

10. Une formulation de poudre thérapeutique selon l'une quelconque des revendications précédentes, qui comprend, en outre, un véhicule, choisi de préférence parmi le groupe comprenant le tréhalose, le raffinose, le mannitol, le sorbitol, le xylitol, l'inositol. le saccharose, le chlorure de sodium et le citrate de sodium.

11. Une formulation de poudre thérapeutique selon l'une quelconque des revendications précédentes, qui comprend, en outre, une quantité stabilisante d'un composé phénolique.

12. Une formulation de poudre thérapeutique selon la revendication 11. qui comprend au moins 3 molécules de composé phénolique/hexamère d'insuline.

13. Une formulation de poudre thérapeutique selon la revendication 11 ou 12, qui comprend du m-crésol ou du phénol ou un mélange de ceux-ci.
